# EUROPEAN PATENT SPECIFICATION

(11) **EP 1 344 775 B1**
(45) Date of publication and mention of the grant of the patent: **29.11.2006**
(21) Application number: 03011148.8
(22) Date of filing: 12.06.1997
(51) Int. Cl.: C07D 239/96, C07C 271/68, C07C 331/28

(54) **Preparation of fungicidal quinazolinones and useful intermediates**
Herstellung von fugizides Chinazolinonen und nützliche Zwischenprodukte
Preparation de quinazolinones fongicides et intermediaires utiles

(30) Priority: 18.06.1996 US 20423 P
(43) Date of publication of application: 17.09.2003
(62) Divisional of application: 97931152.9
(73) Proprietor: E.I. DU PONT DE NEMOURS AND COMPANY, Wilmington, Delaware 19898 (US)
(72) Inventor: Bereznak, James Francis, Aston, Pennsylvania 19014 (US); Marshall, Eric Allen, Elkton, Maryland 21921 (US); Sun, King-Mo, Hockessin, Delaware 19707 (US)
(74) Representative: Beacham, Annabel Rose

(56) References cited:
- WO-A-94/26722
- DEAN W D ET AL: "SYNTHESIS OF 4(3H)-QHINAZOLINONES FROM DERIVATIVES OF METHYL 2-ISOTHIOCYANATOBENZOATE" JOURNAL OF HETEROCYCLIC CHEMISTRY, HETEROCORPORATION. PROVO, US, vol. 19, 1 September 1982 (1982-09-01), pages 1117-1123, XP002045780 ISSN: 0022-152X
- GRAEFE, I. ET AL.: "Synthesis of 3-heteroaryl-4-oxo- 2-thioxo- and 3-heteroaryl-2,4-dioxo- 1,2,3,4-tetrahydroquinazolines" PHARMAZIE (1990), 45(7), 530-1, XP001153577

## Description

### FIELD OF THE INVENTION

This invention relates to compounds and processes for the preparation of fungicidal quinazolinones.

### BACKGROUND OF THE INVENTION

WO 94/26722 generically discloses conversion of anthranilic acids to 2-thioquinazolinediones with isothiocyanates (see also: U.S. 3,755,582). WO 94/26722 discloses that the reaction is preferably performed in the presence of a base such as triethylamine.

WO 94/26722 also discloses conversion of 2-thioquinazolinediones to 2-chloro-4(3H)-quinzolinones using sulfuryl chloride (see also: U.S. 3,867,384). No mention is made of using phosgene for this transformation.

WO 94/26722 and U.S. 3,755,582 generically disclose 2-alkylthio-quinazolinones.

WO 94/26722 generically discloses the condensation of anthranilic acid esters with thiophosgene to form isothiocyanate esters. Similar procedures are disclosed in

### J. Heterocycl. Chem., (1990), 27, 407.

The preparation of 2,4-(1H,3H)-quinazolinediones from anthranilic acid and esters plus isocyanates is cited in *J. Heterocycl. Chem.,* (1982), *19*, 269.

EP-A-712849 discloses a process for the preparation of quinazoline-2,4-diones.

### SUMMARY OF THE INVENTION

This invention provides an advantageous process for preparing quinazolinones of Formula I 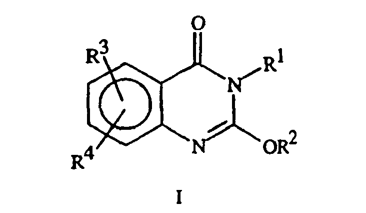 wherein:
R¹ is C₁-C₁₀ alkyl; C₃-C₁₀ alkenyl; C₃-C₁₀ cycloalkyl; C₃-C₁₀ halocycloalkyl; C₄-C₁₀ cycloalkylalkyl; C₄-C₁₀ halocycloalkylalkyl; or C₃-C₁₀ alkynyl;
R² is C₁-C₁₀ alkyl; C₃-C₁₀ alkenyl; C₃-C₁₀ cycloalkyl; C₃-C₁₀ halocycloalkyl; C₄-C₁₀ cycloalkylalkyl; C₄-C₁₀ halocycloalkylalkyl; C₄-C₁₀ cycloalkyl; C₄-C₁₀ halocycloalkyl; or C₃-C₁₀ alkynyl; and R³ and R⁴ are each independently hydrogen or halogen; from compounds containing the moiety IIg

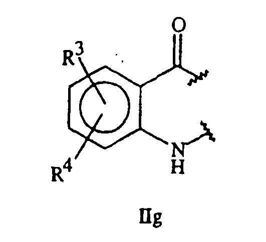

The quinazolinones of Formula I are useful as fungicides and/or intermediates for preparing fungicides. The process for preparing the quinazolinones of Formula I provided herein is characterized by employing a process sequence (D) as described below.

### Process D

A process for preparing compounds of Formula I is provided which comprises treating an anthranilic acid or ester of Formula 1b 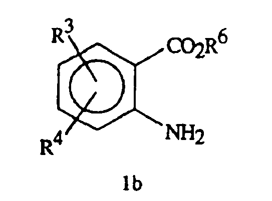 wherein R⁶ is hydrogen or C₁-C₆ alkyl with a compound of Formula III 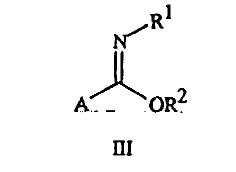 wherein A is Cl or S(C₁-C₆ alkyl) optionally in the presence of an acid or a base, and an inert solvent at a temperature of from about 0 to 100°C, and a pressure of from about 1 to 5 atmospheres (1.013 x 10⁵ to 5.065 x 10⁵ Pa).

This invention further provides a compound of Formula IIIa 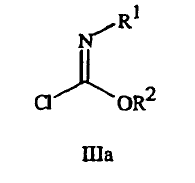 wherein R¹ and R² are C₃ alkyl.

### DETAILED DESCRIPTION OF THE INVENTION

Compounds prepared by the method of this invention can exist as one or more stereoisomer. The various stereoisomers include enantiomers, diastereomers and geometric isomers. One skilled in the art will appreciate that one stereoisomer may be more desirable and how to separate said stereoisomers. Accordingly, the present invention comprises preparation of mixtures, individual stereoisomers, and optically active mixtures of compounds of Formula I.

In the above recitations, the term "alkyl", used either alone or in compound words such as "alkylthio" or "haloalkyl" includes straight-chain or branched alkyl, such as, methyl, ethyl, *n*-propyl, *i*-propyl, or the different butyl, pentyl, hexyl, heptyl, octyl, nonyl, or decyl isomers. The term "1-2 alkyl" indicates that one or two of the available positions for that substituent may be alkyl which are independently selected. "Alkenyl" includes straight-chain or branched alkenes such as vinyl, 1-propenyl, 2-propenyl, and the different butenyl, pentenyl and hexenyl isomers. "Alkenyl" also includes polyenes such as 1,2-propadienyl and 2,4-hexadienyl. "Alkynyl" includes straight-chain or branched alkynes such as ethynyl, 1-propynyl, 2-propynyl and the different butynyl, pentynyl and hexynyl isomers. "Alkynyl" can also include moieties comprised of multiple triple bonds such as 2,5-hexadiynyl. "Cycloalkyl" includes, for example, cyclopropyl, cyclobutyl, cyclopentyl, and cyclohexyl. Examples of "cycloalkylalkyl" include cyclopropylmethyl, cyclopentylethyl, and other cycloalkyl moieties bonded to straight-chain or branched alkyl groups.

The term "halogen" includes fluorine, chlorine, bromine or iodine.

The total number of carbon atoms in a substituent group is indicated by the "Cᵢ-Cⱼ" prefix where i and j are numbers from 1 to 10. For example, C₁-C₃ alkyl designates methyl through propyl; and C₄ alkyl designates the various isomers of an alkyl group containing a total of four carbon atoms.

When a group contains a substituent which can be hydrogen, for example R³ or R⁴, then, when this substituent is taken as hydrogen, it is recognized that this is equivalent to said group being unsubstituted.

The processes of the present invention and methods for the preparation of compounds of Formula I and Formula IIIa (see Scheme 16) are described below. One skilled in the art will recognize when the order of addition of reagents is important in the processes of this invention.

Of note are processes wherein R¹ is C₁-C₁₀ alkyl, C₃-C₁₀ alkenyl, C₄-C₁₀ cycloalkylalkyl, C₄-C₁₀ halocycloalkylalkyl, or C₃-C₁₀ alkynyl; and R² is C₁-C₁₀ alkyl, C₃-C₁₀ alkenyl, C₄-C₁₀ cycloalkylalkyl or C₃-C₁₀ alkynyl. Preferred processes include processes to prepare compounds of Formula I wherein R¹ is C₁-C₃ alkyl, R² is C₁-C₃ alkyl, R³ is Br or I, and R⁴ is H, Br or I.

### Process D- beginning with Compounds of Formula 1b

Compounds of Formula I may be prepared by the sequence beginning with the reaction of the isothiocyanates of Formula 5 with alcohols of Formula 9 to produce intermediates of Formula 10.

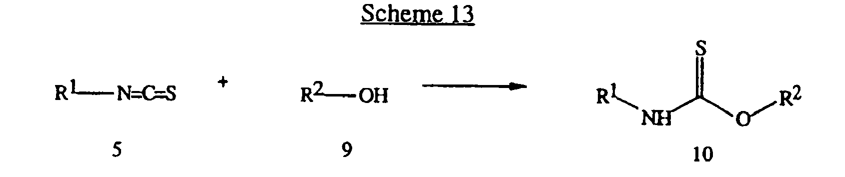

Isothiocyanates of Formula 5 are well-known in the art and can be prepared from the corresponding amines by treatment with thiophosgene (see *e.g., J. Heterocycl. Chem.,* (1990) 27, 407). The conversion of isothiocyanates of Formula 5 to products of Formula 10 has been cited in the art as well (*J. Econ. Entomol.,* (1985)78, 599). The reaction requires prolonged heating of equimolar amounts of an isothiocyanate and alcohol in an inert solvent at elevated temperatures in a pressure bomb apparatus. A preferable alternative for preparing compounds of Formula 10 from isothiocyanates 5 involves reacting the isothiocyanates in neat alcohols of Formula 9 at temperatures ranging from about 50 to 200°C for about 0.5 to 24 h. Following concentration under reduced pressure, the crude material 10 may be purified by distillation at reduced pressure and/or silica gel chromatography; however, crude material of Formula 10 may be carried on without further purification. Materials of Formula 10 are well-known in the art and the skilled practitioner realizes that these compounds can also be prepared by other established means (see JP 5,139,901=CA, *119*:154014v; *Yakhak Hoe Chi,* (1982), 26, 91=*CA*, 97:162320; DE 2353976=*CA*, 83:78658; *Chem. Zvesti,* (1969) 23, 736=*CA*, 73:98568).

Conversion of the compounds of Formula 10 to intermediates of Formula 11 can be accomplished by reacting the compounds of Formula 10 (or anions thereof) with various alkylating agents in suitable solvents at ambient temperatures (Scheme 14). For example, the alkylating agents (R⁵)₃O⁺BF₄⁻ are generally used in halocarbon or hydrocarbon solvents, (R⁵)₂SO₄ in halocarbon, ether, or hydrocarbon solvents, and R⁵-Y (where Y is I, Br, Cl) in alcohol, ether, halocarbon, hydrocarbon, amide, or sulfoxide solvents. Examples of bases which may be employed to optionally generate the anion of compounds of Formula 10 include NaH, NaOR², and K₂CO₃.

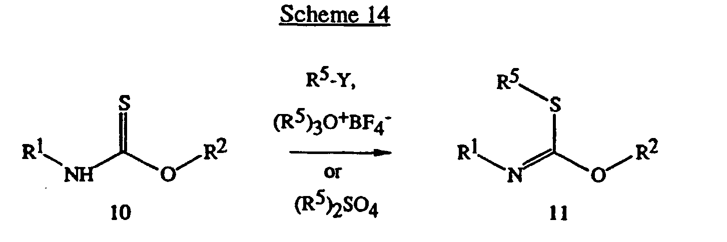

The reaction may be conducted for about 0.5 to 24 h at temperatures ranging from about ambient to 100°C. The mixture may then be subjected to aqueous extraction and concentration under reduced pressure to deliver the crude product of Formula 11 which may be suitable for further transformations. Additional purification, however, may be achieved by distillation at reduced pressure and/or silica gel chromatography.

Reaction of the intermediates of Formula 11 with compounds of Formula 1b yields the targeted compounds of Formula I (Scheme 15). 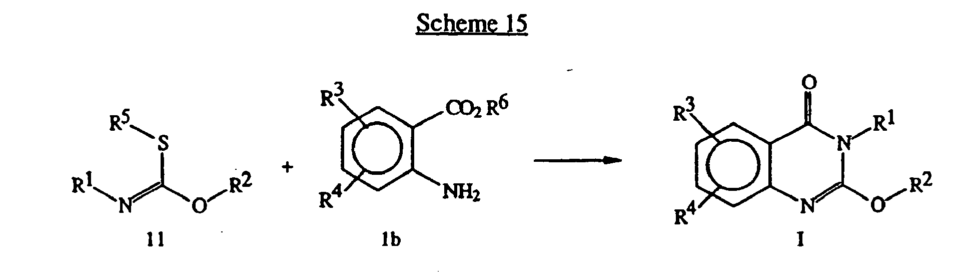 The reaction is conducted in the optional presence of a base (*e.g.-* Et₃N) in an alcohol, ether, or hydrocarbon solvent at temperatures ranging from about 25 to 200°C for about 0.5 to 48 h. Purification of the final products of Formula I can be accomplished by silica-gel chromatography and/or recrystallization.

A preferable alternative for preparing compounds of Formula I from the isothiocyanates of Formula 5 involves the single-vessel preparation and utilization of compounds of Formula 11. The single-vessel preparation of materials similar to compounds of Formula 11 from isothiocyanates such as 5 and alkylating agents R⁵-Y has been documented in the art (*J. Am. Chem. Soc.,* (1983) *105,* 6985 and *Ann*. *Chem.,* (1980) *11*, 1751). Thus, reaction of isothiocyanates 5 in neat alcohols of Formula 9 generates the intermediates of Formula 10 after heating at temperatures between about 50 to 200°C for about 0.5 to 24 h. The resulting mixture is directly treated with base (*e.g.*- M⁺-OR², K₂CO₃, NaH) followed by an alkylating agent R⁵-Y at ambient temperature to afford the compounds of Formula 11 after reaction for about 0.5 to 24 h at 23 to 100°C. Finally, addition of the acid or ester species 1b in the optional presence of the aforementioned catalyst ultimately provides the targeted species of Formula I after further reaction, workup, and purification.

An alternative method for preparing a compound of Formula I is described by Schemes 16, and 17. A specifically preferred compound of Formula I, 6-iodo-3-*n*-propyl-2-*n*-propyloxy-4(3H)-quinazolinone is illustrated.

Contacting propylcarbonimidoyl dichloride, (H. Ulrich, *The Chemistry of Imidoyl Halides, Chapter 2,* Plenum Press, New York, 1968) in a suitable solvent such as methylene chloride with a base such as potassium hydroxide and propanol gives the propyl propylchloroformimidate IIIb, which is then contacted with 5-iodoanthranilic acid, a base such as potassium hydroxide and a catalytic amount of tetra-n-butylammonium iodide to give a 6-iodo-3-n-propyl-2-n-propyloxy-4(3H)-quinazolinone.

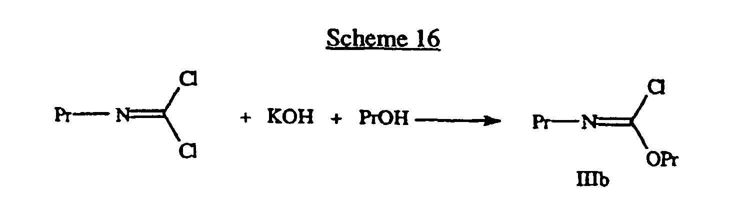

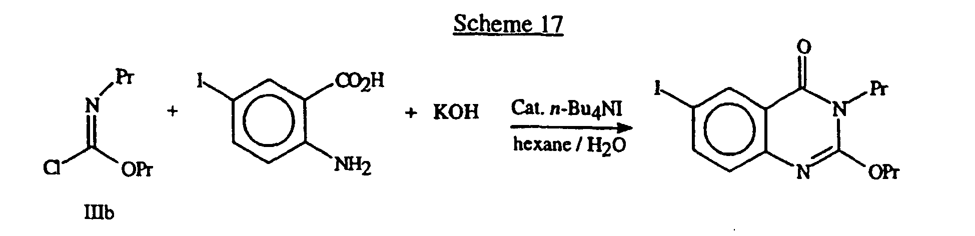

The isopropyl analog of the compound IIIb can be prepared by using the analogous starting materials isopropylcarbonimidoyl dichloride and isopropyl alcohol.

### EXAMPLE I

### Process D: Synthesis of 6-Iodo-2-propoxy-3-propyl-4(3H)-quinazolinone

### Step A: Preparation of O-Propyl propylcarbamothioate

To 10 g (0.099 g) of propyl isothiocyanate was added 50 mL of *n*-propanol. The reaction mixture was heated to reflux for 3 h and cooled to room temperature. An aliquot was removed and concentrated under reduced pressure to give the title compound, ¹H NMR (300 MHz, CDCl₃): δ 0.94-1.02 (m,6H); 1.49-1.83 (m,4H); 3.22 (q), 3.51 (q,2H total); 4.37 (t), 4.45 (t,2H total), 6.25 (br s), 6.78 (br s,1H total). The remaining reaction mixture was concentrated under reduced pressure as above to provide 13.5 g of oil which was used without further purification.

### Step B: Preparation of S-Methyl O-propyl propylcarbonimidothioate

To a solution of 5 g (0.031 moles) of O-propyl propylcarbamothioate in 155 mL of tetrahydrofuran was added 1.37 g (0.034 moles) of 60% NaH at 0-5°C. After allowing the mixture to warm to room temperature, 2.49 mL (0.040 moles) of iodomethane was added and the reaction was stirred for 2 h. The reaction was partitioned between 200 mL each of diethyl ether and saturated aqueous sodium bicarbonate. The organic phase was separated, washed with water and brine, dried over anhydrous sodium sulfate, and concentrated under reduced pressure to deliver 5.9 g of the title compound, which was carried on without further purification, ¹H NMR (400 MHz, CDCl₃): δ 0.91-0.99 (m,6H); 1.56-1.62 (m,2H); 1.68-1.75 (m,2H); 2.38 (s,3H); 3.14 (t,2H); 4.09 (t,2H).

### Step C: Preparation of 6-Iodo-2-propoxy-3-propyl-4(3H)-quinazolinone

To a solution of 0.38 g (1.44 mmoles) of 2-amino-5-iodobenzoic acid and 0.14 g (1.38 mmol) of triethylamine in 7.15 mL of n-propanol was added 0.25 g (1.143 mmol) of S-methyl O-propyl propylcarbonimidothioate. The reaction mixture was stirred at room temperature for 18 h, heated to 60°C for 24 h, and then heated to reflux for 6 h. The reaction was then concentrated under reduced pressure to give about 0.6 g of crude material, which was purified by flash chromatography on silica (93:7 hexanes:ethyl acetate) to give 0.21 g of the title compound, ¹H NMR (400 MHz, CDCl₃): δ 0.96 (t,3H); 1.06 (t,3H); 1.64-1.79 (m,2H); 1.81-1.92 (m,2H); 4.05 (t,2H); 4.43 (t,2H), 7.19 (d,1H); 7.85 (dd,1H); 8.49 (d,1H). In addition, 0.11g of propyl-5-iodoanthranilate was obtained from the flash chromatography, ¹H NMR (400 MHz, CDCl₃): δ 1.02 (t,3H); 1.76-1.86 (m,2H); 4.23 (t,2H); 5.8 (br s,2H); 6.45 (d,1H); 7.45 (dd, 1H); 8.13 (d,1H).

### EXAMPLE 2

### Process D: Synthesis of 6-Iodo-2-propoxy-3-propyl-4(3H)-quinazolinone

### Step A: Preparation of Propyl propylchloroformidate

To a stirred solution of propylcarbonimidoyl dichloride (1.2 g, 8.57 mmol) in dichloromethane (3 mL) cooled to -5-0°C, potassium propoxide (prepared from potassium hydroxide and propanol and removing water by azeotrope) in propanol was added dropwise, keeping the reaction temperature at about 0°C. The transformation of the C=N absorption at 1650 cm⁻¹ for propylcarbonimidoyl dichloride to 1697 cm⁻¹ for propyl propylchloroformimidate was monitored by IR. When the reaction was complete, the reaction mixture was poured into water (10 mL) and extracted with diethyl ether (3 times with 10 mL). The diethyl ether extracts were combined, dried (MgSO₄), and concentrated under reduced pressure to give propyl propylchloroformimidate (1.35 g, 96%, oil; b.p. 61-62°C (1.3 x 10³ Pa). IR (CCl₄): 2966.8, 1697.4, 1186.4 cm⁻¹.

### Step B: Preparation of 6-Iodo-2-propoxy-3-propyl-4(3H)-quinazolinone from 5-Iodoanthranilic acid and Propyl propylchloroformimidate

To a vigorously stirred mixture of 5-iodoanthranilic acid (1 g, 3.8 mmol), potassium hydroxide (0.8 g, 14.3 mmol) and a catalytic amount of tetra-*n*-butylammonium iodide in water (8 mL) at 20°C, propyl propylchloroformimidate (1.37 g, 8.38 mmole) in hexane (4 mL) was added. The reaction was stirred vigorously and the reaction temperature was maintained between 20-25°C with the use of an external water bath. The reaction was monitored by TLC. After 15 min of stirring, hexane (10 mL) was added. The aqueous layer was separated and extracted with hexane (10 mL). The combined hexane layers were washed with HCl (5 mL), saturated aqueous NaHCO₃ (5 mL), and water (5 mL); dried over magnesium sulfate; suction filtered; and concentrated under reduced pressure to an oil which was purified by silica gel column (eluted with ethyl acetate/hexane = 1/9) to provide the product (1.2 g, 85%). Recrystallization from 1-propanol/water gave a crystalline white solid (m.p. 51.5°C). ¹H NMR (in CDCl₃) δ: 0.97 (t,3H), 1.06 (t,3H), 1.66 (m,2H), 1.85 (m,2H), 4.06 (t,2H), 4.43 (t,2H), 7.18 (d,1H), 7.84 (dd,1H), 8.48 (d,1H).

Using the procedures outlined in Schemes 13-17 and Examples 1-2, the compounds of Tables I, Ia, Ib, Ic and Id can be prepared.

**Table I**

| |
|---|
| 3-propyl-2-(propyloxy)-4(3H)-quinazolinone |
| 2-ethoxy-6-iodo-3-propyl-4(3H)-quinazolinone |
| 6-iodo-2-methoxy-3-propyl-4(3H)-quinazolinone |
| 3-ethyl-6-iodo-2-(propyloxy)-4(3H)-quinazolinone |
| 6-iodo-3-methyl-2-(propyloxy)-4(3H)-quinazolinone |
| 6-iodo-2-(2-propenyloxy)-3-(propyl)-4(3H)-quinazolinone |
| 6-iodo-3-(2-propenyl)-2-(propyloxy)-4(3H)-quinazolinone |
| 3-ethyl-1,2-dihydro-6-iodo-2-thioxo-4(3H)-quinazolinone |
| 2-chloro-3-ethyl-6-iodo-4(3H)-quinazolinone |
| 2-chloro-6-iodo-3-methyl-4(3H)-quinazolinone |
| 3-ethyl-6-iodo-2,4-(1H,3H)-quinazolinedione |
| 6-iodo-3-methyl-2,4-(1H,3H)-quinazolinedione |
| 2-(ethylthio)-6-iodo-3-propyl-4(3H)-quinazolinone |
| 3-ethyl-6-iodo-2-(methylthio)-4(3H)-quinazolinone |
| 6,8-diiodo-3-propyl-2-propyloxy-4(3H)-quinazolinone |
| 3-(cyclopropylmethyl)-6-iodo-2-propyloxy-4(3H)-quinazolinone |

**Table Ia**

| | | | |
|---|---|---|---|
| 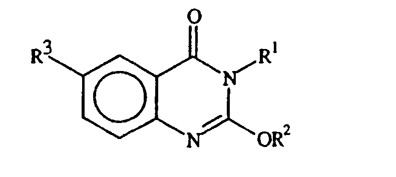 | | | |

| Compound No. | R³ | R¹ | R² |
|---|---|---|---|
| 1 | I | CH₃ | CH₃ |
| 2 | I | CH₂CH₃ | CH₃ |
| 3 | I | CH₂CH₂CH₃ | CH₃ |
| 4 | I | CH₂(CH₂)₂CH₃ | CH₃ |
| 5 | I | CH₃ | CH₂CH₃ |
| 6 | I | CH₂CH₃ | CH₂CH₃ |
| 7 | I | CH₂CH₂CH₃ | CH₂CH₃ |
| 8 | I | CH₃ | CH₂CH₂CH₃ |
| 9 | I | CH₂CH₃ | CH₂CH₂CH₃ |
| 10 | I | CH₃ | CH₂(CH₂)₂CH₃ |
| 11 | Br | CH₃ | CH₃ |
| 12 | Br | CH₂CH₃ | CH₃ |
| 13 | Br | CH₂CH₂CH₃ | CH₃ |
| 14 | Br | CH₂(CH₂)₂CH₃ | CH₃ |
| 15 | Br | CH₃ | CH₂CH₃ |
| 16 | Br | CH₂CH₃ | CH₂CH₃ |
| 17 | Br | CH₂CH₂CH₃ | CH₂CH₃ |
| 18 | Br | CH₃ | CH₂CH₂CH₃ |
| 19 | Br | CH₂CH₃ | CH₂CH₂CH₃ |
| 20 | Br | CH₃ | CH₂(CH₂)₂CH₃ |
| 21 | I | CH(CH₃)₂ | CH₃ |
| 22 | I | CH(CH₃)₂ | CH₂CH₃ |
| 23 | I | CH(CH₃)₂ | CH(CH₃)₂ |
| 24 | I | CH₃ | CH(CH₃)₂ |
| 25 | I | CH₂CH₃ | CH(CH₃)₂ |
| 26 | I | CH(CH₃)₂ | CH(CH₃)₂ |
| 27 | I | C(CH₃)₃ | CH₃ |
| 28 | I | C(CH₃)₃ | CH₂CH₃ |
| 29 | I | C(CH₃)₃ | CH(CH₃)₂ |
| 30 | I | C(CH₃)₃ | C(CH₃)₃ |
| 31 | I | CH₃ | C(CH₃)₃ |
| 32 | I | CH₂CH₃ | C(CH₃)₃ |
| 33 | I | CH(CH₃)₂ | C(CH₃)₃ |
| 34 | I | C(CH₃)₃ | C(CH₃)₃ |
| 35 | Br | CH(CH₃)₂ | CH₃ |
| 36 | Br | CH(CH₃)₂ | CH₂CH₃ |
| 37 | Br | CH(CH₃)₂ | CH(CH₃)₂ |
| 38 | Br | CH₃ | CH(CH₃)₂ |
| 39 | Br | CH₂CH₃ | CH(CH₃)₂ |
| 40 | Br | CH(CH₃)₂ | CH(CH₃)₂ |
| 41 | Br | C(CH₃)₃ | CH₃ |
| 42 | Br | C(CH₃)₃ | CH₂CH₃ |
| 43 | Br | C(CH₃)₃ | CH(CH₃)₂ |
| 44 | Br | C(CH₃)₃ | C(CH₃)₃ |
| 45 | Br | CH₃ | C(CH₃)₃ |
| 46 | Br | CH₂CH₃ | C(CH₃)₃ |
| 47 | Br | CH(CH₃)₂ | C(CH₃)₃ |
| 48 | Br | C(CH₃)₃ | C(CH₃)₃ |
| 49 | I | CH₂CH(CH₃)₂ | CH₃ |
| 50 | I | CH₂CH(CH₃)₂ | CH₂CH₃ |
| 51 | I | CH₂CH(CH₃)₂ | CH(CH₃)₂ |
| 52 | I | CH₃ | CH₂CH(CH₃)₂ |
| 53 | I | CH₂CH₃ | CH₂CH(CH₃)₂ |
| 54 | I | CH(CH₃)₂ | CH₂CH(CH₃)₂ |
| 55 | I | CH₂CH(CH₃)₂ | CH₂CH(CH₃)₂ |
| 56 | I | CH₂C(CH₃)₃ | CH₃ |
| 57 | I | CH₂C(CH₃)₃ | CH₂CH₃ |
| 58 | I | CH₂C(CH₃)₃ | CH(CH₃)₂ |
| 59 | I | CH₂C(CH₃)₃ | C(CH₃)₃ |
| 60 | I | CH₃ | CH₂C(CH₃)₃ |
| 61 | I | CH₂CH₃ | CH₂C(CH₃)₃ |
| 62 | I | CH(CH₃)₂ | CH₂C(CH₃)₃ |
| 63 | I | C(CH₃)₃ | CH₂C(CH₃)₃ |
| 64 | Br | CH₂CH(CH₃)₂ | CH₃ |
| 65 | Br | CH₂CH(CH₃)₂ | CH₂CH₃ |
| 66 | Br | CH₂CH(CH₃)₂ | CH(CH₃)₂ |
| 67 | Br | CH₃ | CH₂CH(CH₃)₂ |
| 68 | Br | CH₂CH₃ | CH₂CH(CH₃)₂ |
| 69 | Br | CH(CH₃)₂ | CH₂CH(CH₃)₂ |
| 70 | Br | CH₂CH(CH₃)₂ | CH₂CH(CH₃)₂ |
| 71 | Br | CH₂C(CH₃)₃ | CH₃ |
| 72 | Br | CH₂C(CH₃)₃ | CH₂CH₃ |
| 73 | Br | CH₂C(CH₃)₃ | CH(CH₃)₂ |
| 74 | Br | CH₂C(CH₃)₃ | C(CH₃)₃ |
| 75 | Br | CH₃ | CH₂C(CH₃)₃ |
| 76 | Br | CH₂CH₃ | CH₂C(CH₃)₃ |
| 77 | Br | CH(CH₃)₂ | CH₂C(CH₃)₃ |
| 78 | Br | C(CH₃)₃ | CH₂C(CH₃)₃ |
| 79 | I | 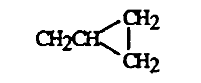 | CH₃ |
| 80 | I | 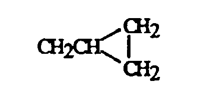 | CH₂CH₃ |
| 81 | I | 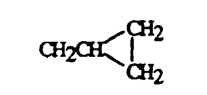 | CH₂CH₂CH₃ |
| 82 | I | CH₃ | 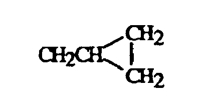 |
| 83 | I | CH₂CH₃ | 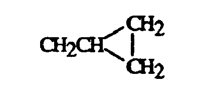 |
| 84 | I | CH₂CH₂ CH₃ | 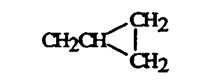 |
| 85 | I | 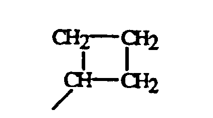 | CH₃ |
| 86 | I | 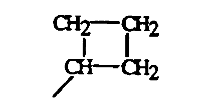 | CH₂CH₃ |
| 87 | I | 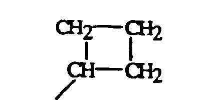 | CH₂CH₂CH₃ |
| 88 | I | CH₃ | 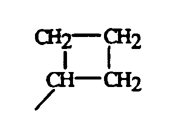 |
| 89 | I | CH₂CH₃ | 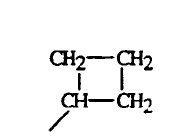 |
| 90 | I | CH₂CH₂CH₃ | 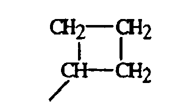 |
| 91 | I | 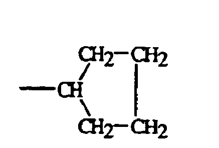 | CH₃ |
| 92 | I | 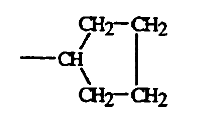 | CH₂CH₃ |
| 93 | I | 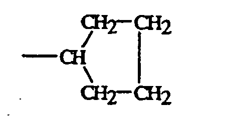 | CH₂CH₂CH₃ |
| 94 | I | CH₃ | 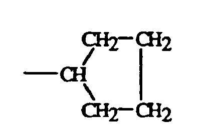 |
| 95 | I | CH₂CH₃ | 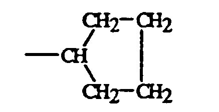 |
| 96 | I | CH₂CH₂CH3 | 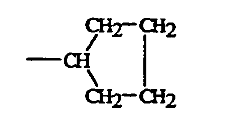 |

**Table Ib**

| | | | | |
|---|---|---|---|---|
| 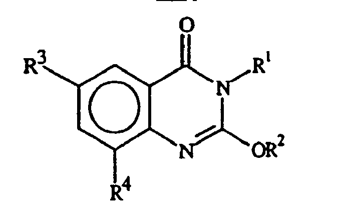 | | | | |

| Compound No. | R³ | R⁴ | R¹ | R² |
|---|---|---|---|---|
| 97 | I | I | CH₃ | CH₃ |
| 98 | I | I | CH₂CH₃ | CH₃ |
| 99 | I | I | CH₂CH₂CH₃ | CH₃ |
| 100 | I | I | CH₂(CH₂)₂CH₃ | CH₃ |
| 101 | I | I | CH₃ | CH₂CH₃ |
| 102 | I | I | CH₂CH₃ | CH₂CH₃ |
| 103 | I | I | CH₂CH₂CH₃ | CH₂CH₃ |
| 104 | I | I | CH₃ | CH₂CH₂CH₃ |
| 105 | I | I | CH₂CH₃ | CH₂CH₂CH₃ |
| 106 | I | I | CH₃ | CH₂(CH₂)₂CH₃ |
| 107 | Br | Br | CH₃ | CH₃ |
| 108 | Br | Br | CH₂CH₃ | CH₃ |
| 109 | Br | Br | CH₂CH₂CH₃ | CH₃ |
| 110 | Br | Br | CH₂(CH₂)₂CH₃ | CH₃ |
| 111 | Br | Br | CH₃ | CH₂CH₃ |
| 112 | Br | Br | CH₂CH₃ | CH₂CH₃ |
| 113 | Br | Br | CH₂CH₂CH₃ | CH₂CH₃ |
| 114 | Br | Br | CH₃ | CH₂CH₂CH₃ |
| 115 | Br | Br | CH₂CH₃ | CH₂CH₂CH₃ |
| 116 | Br | Br | CH₃ | CH₂(CH₂)₂CH₃ |
| 117 | I | Br | CH₃ | CH₃ |
| 118 | Br | I | CH₃ | CH₃ |
| 119 | I | Br | CH₃ | CH₂CH₂CH₃ |
| 120 | I | I | CH(CH₃)₂ | CH₃ |
| 121 | I | I | CH(CH₃)₂ | CH₂CH₃ |
| 122 | I | I | CH(CH₃)₂ | CH(CH₃)₂ |
| 123 | I | I | CH₃ | CH(CH₃)₂ |
| 124 | I | I | CH₂CH₃ | CH(CH₃)₂ |
| 125 | I | I | CH(CH₃)₂ | CH(CH₃)₂ |
| 126 | I | I | C(CH₃)₃ | CH₃ |
| 127 | I | I | C(CH₃)₃ | CH₂CH₃ |
| 128 | I | I | C(CH₃)₃ | CH(CH₃)₂ |
| 129 | I | I | C(CH₃)₃ | C(CH₃)₃ |
| 130 | I | I | CH₃ | C(CH₃)₃ |
| 131 | I | I | CH₂CH₃ | C(CH₃)₃ |
| 132 | I | I | CH(CH₃)₂ | C(CH₃)₃ |
| 133 | I | I | C(CH₃)₃ | C(CH₃)₃ |
| 134 | Br | Br | CH(CH₃)₂ | CH₃ |
| 135 | Br | Br | CH(CH₃)₂ | CH₂CH₃ |
| 136 | Br | Br | CH(CH₃)₂ | CH(CH₃)₂ |
| 137 | Br | Br | CH₃ | CH(CH₃)₂ |
| 138 | Br | Br | CH₂CH₃ | CH(CH₃)₂ |
| 139 | Br | Br | CH(CH₃)₂ | CH(CH₃)₂ |
| 140 | Br | Br | C(CH₃)₃ | CH₃ |
| 141 | Br | Br | C(CH₃)₃ | CH₂CH₃ |
| 142 | Br | Br | C(CH₃)₃ | CH(CH₃)₂ |
| 143 | Br | Br | C(CH₃)₃ | C(CH₃)₃ |
| 144 | Br | Br | CH₃ | C(CH₃)₃ |
| 145 | Br | Br | CH₂CH₃ | C(CH₃)₃ |
| 146 | Br | Br | CH(CH₃)₂ | C(CH₃)₃ |
| 147 | Br | Br | C(CH₃)₃ | C(CH₃)₃ |
| 148 | I | I | CH₂CH(CH₃)₂ | CH₃ |
| 149 | I | I | CH₂CH(CH₃)₂ | CH₂CH₃ |
| 150 | I | I | CH₂CH(CH₃)₂ | CH(CH₃)₂ |
| 151 | I | I | CH₃ | CH₂CH(CH₃)₂ |
| 152 | I | I | CH₂CH₃ | CH₂CH(CH₃)₂ |
| 153 | I | I | CH(CH₃)₂ | CH₂CH(CH₃)₂ |
| 154 | I | I | CH₂CH(CH₃)₂ | CH₂CH(CH₃)₂ |
| 155 | I | I | CH₂C(CH₃)₃ | CH₃ |
| 156 | I | I | CH₂C(CH₃)₃ | CH₂CH₃ |
| 157 | I | I | CH₂C(CH₃)₃ | CH(CH₃)₂ |
| 158 | I | I | CH₂C(CH₃)₃ | C(CH₃)₃ |
| 159 | I | I | CH₃ | CH₂C(CH₃)₃ |
| 160 | I | I | CH₂CH₃ | CH₂C(CH₃)₃ |
| 161 | I | I | CH(CH₃)₂ | CH₂C(CH₃)₃ |
| 162 | I | I | C(CH₃)₃ | CH₂C(CH₃)₃ |
| 163 | Br | Br | CH₂CH(CH₃)₂ | CH₃ |
| 164 | Br | Br | CH₂CH(CH₃)₂ | CH₂CH₃ |
| 165 | Br | Br | CH₂CH(CH₃)₂ | CH(CH₃)₂ |
| 166 | Br | Br | CH₃ | CH₂CH(CH₃)₂ |
| 167 | Br | Br | CH₂CH₃ | CH₂CH(CH₃)₂ |
| 168 | Br | Br | CH(CH₃)₂ | CH₂CH(CH₃)₂ |
| 169 | Br | Br | CH₂CH(CH₃)₂ | CH₂CH(CH₃)₂ |
| 170 | Br | Br | CH₂C(CH₃)₃ | CH₃ |
| 171 | Br | Br | CH₂C(CH₃)₃ | CH₂CH₃ |
| 172 | Br | Br | CH₂C(CH₃)₃ | CH(CH₃)₂ |
| 173 | Br | Br | CH₂C(CH₃)₃ | C(CH₃)₃ |
| 174 | Br | Br | CH₃ | CH₂C(CH₃)₃ |
| 175 | Br | Br | CH₂CH₃ | CH₂C(CH₃)₃ |
| 176 | Br | Br | CH(CH₃)₂ | CH₂C(CH₃)₃ |
| 177 | Br | Br | C(CH₃)₃ | CH₂C(CH₃)₃ |
| 178 | I | I | 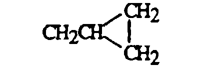 | CH₃ |
| 179 | I | I | 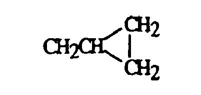 | CH₂CH₃ |
| 180 | I | I | 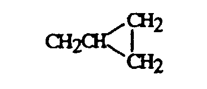 | CH₂CH₂CH₃ |
| 181 | I | I | CH₃ | 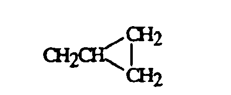 |
| 182 | I | I | CH₂CH₃ | 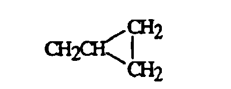 |
| 183 | I | I | CH₂CH₂CH₃ | 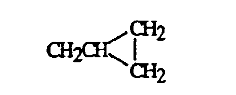 |
| 184 | I | I | 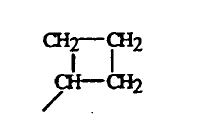 | CH₃ |
| 185 | I | I | 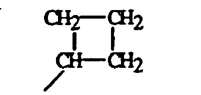 | CH₂CH₃ |
| 186 | I | I | 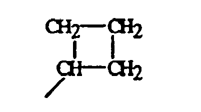 | CH₂CH₂CH₃ |
| 187 | I | I | CH₃ | 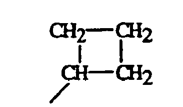 |
| 188 | I | I | CH₂CH₃ | 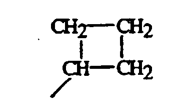 |
| 189 | I | I | CH₂CH₂CH₃ | 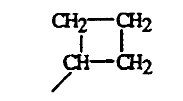 |
| 190 | I | I | 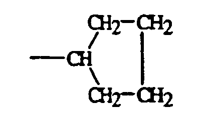 | CH₃ |
| 191 | I | I | 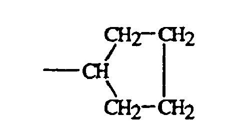 | CH₂CH₃ |
| 192 | I | I | 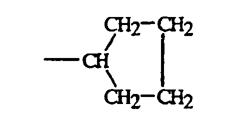 | CH₂CH₂CH₃ |
| 193 | I | I | CH₃ | 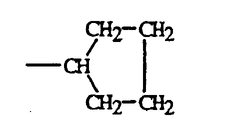 |
| 194 | I | I | CH₂CH₃ | 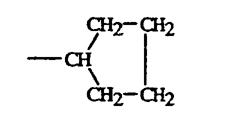 |
| 195 | I | I | CH₂CH₂CH₃ | 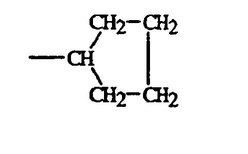 |

**Table Ic**

| | | | |
|---|---|---|---|
| 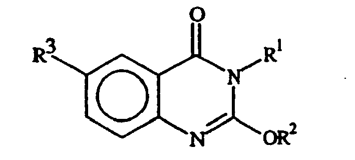 | | | |

| Compound No. | R³ | R¹ | R² |
|---|---|---|---|
| 196 | I | CH₃ | CH=CH₂ |
| 197 | I | CH₂CH₃ | CH=CH₂ |
| 198 | I | CH₂CH₂CH₃ | CH=CH₂ |
| 199 | I | CH₃ | CH₂CH=CH₂ |
| 200 | I | CH₂CH₃ | CH₂CH=CH₂ |
| 201 | I | CH₂CH₂CH₃ | CH₂CH=CH₂ |
| 202 | I | CH=CH₂ | CH₃ |
| 203 | I | CH₂CH=CH₂ | CH₃ |
| 204 | I | CH=CH₂ | CH₂CH₃ |
| 205 | I | CH₂CH=CH₂ | CH₂CH₃ |
| 206 | I | CH=CH₂ | CH₂CH₂CH₃ |
| 207 | I | CH₂CH=CH₂ | CH₂CH₂CH₃ |
| 208 | I | CH=CH₂ | CH=CH₂ |
| 209 | I | CH=CH₂ | CH₂CH=CH₂ |
| 210 | I | CH₂CH=CH₂ | CH=CH₂ |
| 211 | I | CH₂CH=CH₂ | CH₂CH=CH₂ |
| 212 | Br | CH₃ | CH=CH₂ |
| 213 | Br | CH₂CH₃ | CH=CH₂ |
| 214 | Br | CH₂CH₂CH₃ | CH=CH₂ |
| 215 | Br | CH₃ | CH₂CH=CH₂ |
| 216 | Br | CH₂CH₃ | CH₂CH=CH₂ |
| 217 | Br | CH₂CH₂CH₃ | CH₂CH=CH₂ |
| 218 | Br | CH=CH₂ | CH₃ |
| 219 | Br | CH₂CH=CH₂ | CH₃ |
| 220 | Br | CH=CH₂ | CH₂CH₃ |
| 221 | Br | CH₂CH=CH₂ | CH₂CH₃ |
| 222 | Br | CH=CH₂ | CH₂CH₂CH₃ |
| 223 | Br | CH₂CH=CH₂ | CH₂CH₂CH₃ |
| 224 | Br | CH=CH₂ | CH=CH₂ |
| 225 | Br | CH=CH₂ | CH₂CH=CH₂ |
| 226 | Br | CH₂CH=CH₂ | CH=CH₂ |
| 227 | Br | CH₂CH=CH₂ | CH₂CH=CH₂ |

**Table Id**

| | | | | |
|---|---|---|---|---|
| 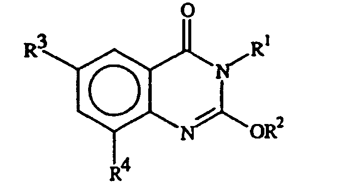 | | | | |

| Compound No. | R³ | R⁴ | R¹ | R² |
|---|---|---|---|---|
| 228 | I | I | CH₃ | CH=CH₂ |
| 229 | I | I | CH₂CH₃ | CH=CH₂ |
| 230 | I | I | CH₂CH₂CH₃ | CH=CH₂ |
| 231 | I | I | CH₃ | CH₂CH=CH₂ |
| 232 | I | I | CH₂CH₃ | CH₂CH=CH₂ |
| 233 | I | I | CH₂CH₂CH₃ | CH₂CH=CH₂ |
| 234 | I | I | CH=CH₂ | CH₃ |
| 235 | I | I | CH₂CH=CH₂ | CH₃ |
| 236 | I | I | CH=CH₂ | CH₂CH₃ |
| 237 | I | I | CH₂CH=CH₂ | CH₂CH₃ |
| 238 | I | I | CH=CH₂ | CH₂CH₂CH₃ |
| 239 | I | I | CH₂CH=CH₂ | CH₂CH₂CH₃ |
| 240 | I | I | CH=CH₂ | CH=CH₂ |
| 241 | I | I | CH=CH₂ | CH₂CH=CH₂ |
| 242 | I | I | CH₂CH=CH₂ | CH=CH₂ |
| 243 | I | I | CH₂CH=CH₂ | CH₂CH=CH₂ |
| 244 | Br | Br | CH₃ | CH=CH₂ |
| 245 | Br | Br | CH₂CH₃ | CH=CH₂ |
| 246 | Br | Br | CH₂CH₂CH₃ | CH=CH₂ |
| 247 | Br | Br | CH₃ | CH₂CH=CH₂ |
| 248 | Br | Br | CH₂CH₃ | CH₂CH=CH₂ |
| 249 | Br | Br | CH₂CH₂CH₃ | CH₂CH=CH₂ |
| 250 | Br | Br | CH=CH₂ | CH₃ |
| 251 | Br | Br | CH₂CH=CH₂ | CH₃ |
| 252 | Br | Br | CH=CH₂ | CH₂CH₃ |
| 253 | Br | Br | CH₂CH=CH₂ | CH₂CH₃ |
| 254 | Br | Br | CH=CH₂ | CH₂CH₂CH₃ |
| 255 | Br | Br | CH₂CH=CH₂ | CH₂CH₂CH₃ |
| 256 | Br | Br | CH=CH₂ | CH=CH₂ |
| 257 | Br | Br | CH=CH₂ | CH₂CH=CH₂ |
| 258 | Br | Br | CH₂CH=CH₂ | CH=CH₂ |
| 259 | Br | Br | CH₂CH=CH₂ | CH₂CH=CH₂ |
| 260 | I | Br | CH₃ | CH₂CH=CH₂ |
| 261 | Br | I | CH₃ | CH₂CH=CH₂ |

## Claims

1. A process for preparing a quinazolinone of Formula I 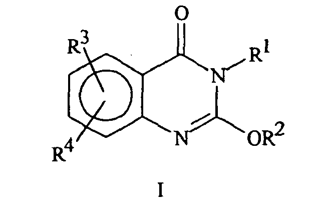 wherein:
R¹ is C₁-C₁₀ alkyl; C₃-C₁₀ alkenyl; C₃-C₁₀ cycloalkyl; C₃-C₁₀ halocycloalkyl; C₄-C₁₀ cycloalkylalkyl; C₄-C₁₀ halocycloalkylalkyl; or C₃-C₁₀ alkynyl;
R² is C₁-C₁₀ alkyl; C₃-C₁₀ alkenyl; C₃-C₁₀ cycloalkyl; C₃-C₁₀ halocycloalkyl; C₄-C₁₀ cycloalkylalkyl; C₄-C₁₀ halocycloalkylalkyl; C₄-C₁₀ cycloalkyl; C₄-C₁₀ halocycloalkyl; or C₃-C₁₀ alkynyl; and R³ and R⁴ are each independently hydrogen or halogen; from compounds containing the moiety IIg 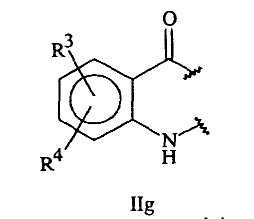
**characterized by** employing a process sequence comprising treating an anthranilic acid or ester of Formula 1b 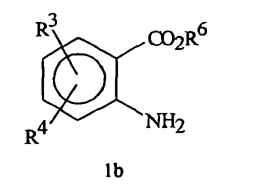 wherein R⁶ is hydrogen or C₁-C₆ alkyl with a compound of Formula III 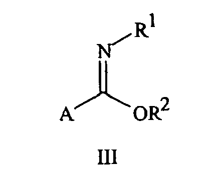 wherein A is Cl or S(C₁-C₆ alkyl) optionally in the presence of an acid or a base, and an inert solvent at a temperature of from 0 to 100°C, and a pressure of from 1 to 5 atmospheres:

2. A process of Claim 1 wherein R¹ is C₁-C₃ alkyl, R² is C₁-C₃ alkyl, R³ is Br or I, and R⁴ is H, Br or I.

3. The process of Claim 2 wherein 2-amino-5-iodobenzoic acid is treated with S-methyl, O-propyl-propylcarbonimidothioate.

4. A compound of Formula IIIa 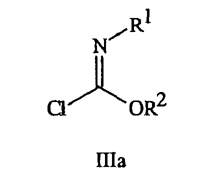 wherein R¹ and R² are C₃ alkyl.

## Patentansprüche

1. Verfahren zur Herstellung eines Chinazolinons der Formel I 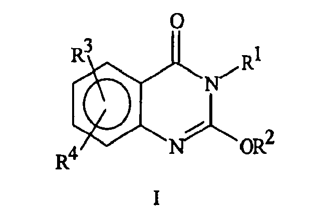 worin:
R¹ für C₁-C₁₀-Alkyl; C₃-C₁₀-Alkenyl; C₃-C₁₀-Cycloalkyl; C₃-C₁₀-Halocycloalkyl; C₄-C₁₀-Cycloalkylalkyl; C₄-C₁₀-Halocycloalkylalkyl; oder C₃-C₁₀-Alkinyl steht;
R² für C₁-C₁₀-Alkyl; C₃-C₁₀-Alkenyl; C₃-C₁₀-Cycloalkyl; C₃-C₁₀-Halocycloalkyl; C₄-C₁₀-Cycloalkylalkyl; C₄-C₁₀-Halocycloalkylalkyl; C₄-C₁₀-Cycloalkyl; C₄-C₁₀-Halocycloalkyl; oder C₃-C₁₀-Alkinyl steht; und R³ und R⁴ jeweils unabhängig für Wasserstoff oder Halogen stehen; aus Verbindungen, enthaltend die Komponente IIg 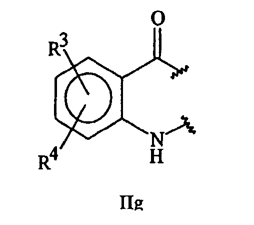 **gekennzeichnet durch** den Einsatz einer Verfahrensfolge, umfassend die Behandelung einer Anthranilsäure oder eines -esters der Formel 1b 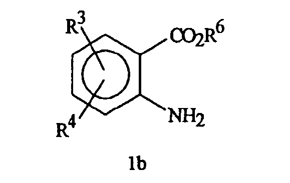
worin R⁶ für Wasserstoff oder C₁-C₆-Alkyl steht, mit einer Verbindung der Formel III 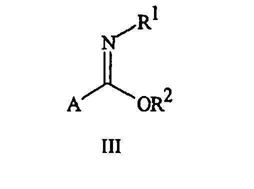 worin A für Cl oder S(C₁-C₆-Alkyl) steht, optional in Anwesenheit einer Säure oder Base, und einem inerten Lösungsmittel bei einer Temperatur von 0 bis 100 °C und einem Druck von 1 bis 5 Atmosphären.

2. Verfahren nach Anspruch 1, worin R¹ für C₁-C₃-Alkyl steht, R² für C₁-C₃-Alkyl steht, R³ für Br oder I steht und R⁴ für H, Br oder I steht.

3. Verfahren nach Anspruch 2, worin 2-Amino-5-iodbenzoesäure mit S-Methyl-O-propyl-propylcarbonimidthioat behandelt wird.

4. Verbindung der Formel IIIa 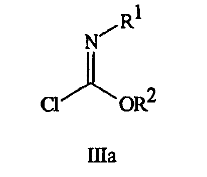 worin R¹ und R² für C₃-Alkyl stehen.

## Revendications

1. Procédé pour la préparation d'une quinazolinone de la Formule I: 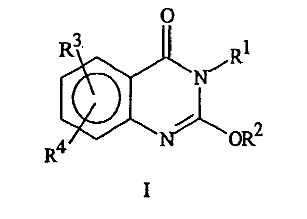 dans laquelle:
R¹ est un groupe alkyle C₁-C₁₀; alcényle C₃-C₁₀; cycloalkyle C₃-C₁₀; halocycloalkyle C₃-C₁₀; cycloalkylalkyle C₄-C₁₀; halocycloalkylalkyle C₄-C₁₀; ou alcynyle C₃-C₁₀;
R² est un groupe alkyle C₁-C₁₀; alcényle C₃-C₁₀; cycloalkyle C₃-C₁₀; halocycloalkyle C₃-C₁₀; cycloalkylalkyle C₄-C₁₀; halocycloalkylalkyle C₄-C₁₀; cycloalkyle C₄-C₁₀; halocycloalkyle C₄-C₁₀; ou alcynyle C₃-C₁₀; et R³ et R⁴ sont chacun indépendamment un hydrogène ou un halogène; à partir de composés contenant la fraction IIg: 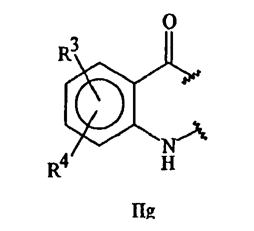
**caractérisé par** l'emploi d'une séquence de procédé comprenant le traitement d'un acide ou ester anthranilique de la Formule 1b: 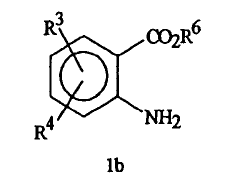 dans laquelle R⁶ est un hydrogène ou un groupe alkyle C₁-C₆ avec un composé de la Formule III: 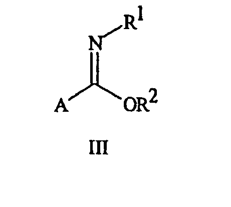 dans laquelle A est Cl ou un groupe S(alkyle C₁-C₆) éventuellement en présence d'un acide ou d'une base, et un solvant inerte à une température de 0 à 100°C et une pression de 1 à 5 atmosphères.

2. Procédé suivant la revendication 1, dans lequel R¹ est un groupe alkyle C₁-C₃, R² est un groupe alkyle C₁-C₃, R³ est Br ou I, et R⁴ est H, Br ou I.

3. Procédé suivant la revendication 2, dans lequel l'acide 2-amino-5-iodobenzoïque est traité avec le S-méthyl-O-propyl-propylcarbonimidothioate.

4. Composé de la Formule IIIa: 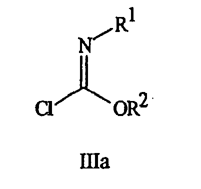 dans laquelle R¹ et R² sont un groupe alkyle C₃.
